Europäisches Patentamt

European Patent Office

Office européen des brevets

(11) Publication number: **0 190 362 B1**

# EUROPEAN PATENT SPECIFICATION

(45) Date of publication of patent specification: **22.04.92** (51) Int. Cl.⁵: **C12N 5/04**, C12P 1/00

(21) Application number: **85904019.8**

(22) Date of filing: **09.08.85**

(86) International application number:
**PCT/JP85/00446**

(87) International publication number:
**WO 86/01225 (27.02.86 86/05)**

(54) PROCESS FOR TREATING CELLS.

(30) Priority: **09.08.84 JP 165671/84**

(43) Date of publication of application:
**13.08.86 Bulletin 86/33**

(45) Publication of the grant of the patent:
**22.04.92 Bulletin 92/17**

(84) Designated Contracting States:
**DE FR GB IT NL**

(56) References cited:
**EP-A- 0 004 223**
**EP-A- 0 027 662**
**US-A- 4 394 448**

**CHEMICAL ABSTRACTS, vol. 97, 1982, page
327, abstract no. 36258g, Columbus, Ohio,
US; M. TSUZUKI et al.: "Role of carbonic
anhydrase in photosynthesis of higher
plants: a posible limiting step in carbon
dioxide transport", & PHOTOSYNTH. PROC.
INT. CONGR., 5th 1980 (PUb. 1981), 4, 483-92**

(73) Proprietor: **KABUSHIKI KAISYA ADVANCE**
**5-7, Nihonbashi Kobuna-cho**
**Chuo-ku Tokyo 103(JP)**

(72) Inventor: **OKABE, Keiichiro**
**8-30, Seijo Setagaya-ku**
**Tokyo 157(JP)**
Inventor: **KAWAI, Yasuo**
**2-8-12, Moridai Atsugi-shi**
**Kanagawa 243(JP)**

(74) Representative: **Ellis, John Clifford Holgate et
al**
**MEWBURN ELLIS & CO. 2/3 Cursitor Street
London EC4A 1BO(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

CHEMICAL ABSTRACTS, vol. 93, 1980, page 397, abstract no. 40355w, Columbus, Ohio, US; K. OKABE et al.: "Effects of carbonic anhydrase on ribulose 1,5-bisphosphate carboxylase and oxygenase", & FEBS LETT. 1980, 114(1), 142-4

CHEMICAL ABSTRACTS, vol. 87, 1977, page 136, abstract no. 34772q, Columbus, Ohio, US; J. GUTKNECHT et al.: "Diffusion of carbon dioxide through lipid bilayer membranes. Effects of carbonic anhydrase, bicarbonate, and unstirred layers", & J. GEN. PHYSIOL. 1977, 69(6), 779-94

CHEMICAL ABSTRACTS, vol. 88, 1978, page 115, abstract no. 16673z, Columbus, Ohio, US; R.L. DORMER et al.: "Studies on the entrapment of the calcium-activated phtoprotein obelin and carbonic anhydrase in liposomes and their interaction with isolated adipocytes", & BIOCHEM. SOC. TRANS. 1977, 5(4), 1151-4

CHEMICAL ABSTRACTS, vol. 96, 1982, page 358, abstract no. 82914v, Columbus, Ohio, US; YU. S. NASYROV et al.: "Genetic modification of the carbon dioxide carboxylation reactions as a factor improving efficiency of photosynthesis", & INDIAN J. PLANT PHYSIOL. 1981, 24(1), 26-36

CHEMICAL ABSTRACTS, vol. 87, 1977, page 351, abstract no. 130430x, Columbus, Ohio, US; D.N. MOSS et al.: "Improvement of plant photosynthesis through genetic engineering", & CLEAN FUELS BIOMASS WASTES, SYMP. PAP. 1977, 63-71

SCIENCE, vol. 219, 11th February 1983, pages 671-676; K.A. BARTON et al.: "Prospects in plant genetic engineering"

WORLD BIOTECH. REPORTS, Europe Conf., May 1984, London, vol. 2, pages A3-A6, Online Publications, Pinner, GB; K. ANDERSEN et al.: "Genetic engineering for enhancing photosynthetic carbon dioxide fixation efficiency of crop plants"

CHEMICAL ABSTRACTS, vol. 98, 1983, page 169, abstract no. 192672p, Columbus, Ohio, US; P.J. CURTIS: "Cloning of mouse carbonic anhydrase mRNA and its induction in mouse erythroleukemic cells", & J. BIOL. CHEM. 1983, 258(7), 4459-63

EP 0 190 362 B1

**Description**

This invention relates to a cellular engineering method for improving the cellular metabolic processes of animals, plants, algae, and other microorganisms.

More particularly, this invention relates to a cellular engineering method for improving the photosynthetic capacity of C3 plants such as the tomato, tobacco, spinach, soybean, rice, and wheat. This invention resides in a cellular engineering method whereby the rate limiting step(s) in the photosynthetic $CO_2$ transporting (i.e., from the outside to the $CO_2$ fixation sites) process in C3 plants, cytosol, and/or cytomembrane, is (are) improved to increase the efficiency of the photosynthetic $CO_2$ fixation by the introduction of a carbonic anhydrase activity into the intracellular space, cytosol, and/or cytomembrane.

BACKGROUND ART

The concentration of environmental $CO_2$ is one of the eminent rate limiting factors for plant photosynthetic $CO_2$ fixation, on which world food production is primarily based. This is apparent from the fact that an increase in the environmental $CO_2$ concentration (i.e., usually 0.03%) results in an increase of the photosynthetic rate in many species and, therefore, the rate of supply of photosynthetic substrate $CO_2$ to the carboxylation site is not optimum for the maximum photosynthetic performance. In practice, the so-called $CO_2$ feeding technique has been successfully developed for tomato intensive cultures in green houses in Japan, to increase photosynthesis.

DISCLOSURE OF THE INVENTION

Accordingly, the object of this invention is to provide a method of increasing the cellular $CO_2$ fixation capacity by the introduction of a carbonic anhydrase activity into the cytosol and/or cytomembrane of animals, plants, algae, and other microorganisms.

That is, this invention provides a cellular engineering method for improvement of the $CO_2$ fixation capacity of the cells by a direct or genetical introduction of carbonic anhydrase [EC 4.2.1.1] activity into the intracellular cytosol and/or cytomembrane.

BRIEF DESCRIPTION OF THE DRAWINGS

Figures 1 and 2 are schematic diagrams showing the intracellular concentration gradient of photosynthetic substrate inorganic carbon during photosynthesis in the conventional C3 plant and in improved C3 plant types;

Fig. 3 is a graph showing the time course of liposome mediated introduction of carbonic anhydrase into cotyledonous protoplasts in Example 1;

Fig. 4 is a graph showing the photosynthetic efficiencies of cotyledonous protoplasts treated with carbonic anhydrase encapsulated and control liposomes in Example 1;

Fig. 5 is a graph showing the degree of enhancement of the photosynthetic $CO_2$ fixation rate by the introduction of carbonic anhydrase into cotyledonous protoplasts by the mediation of liposomes in Example 1;

Fig. 6 is a graph showing photosynthetic $O_2$ evolution rates in relation to substrate bicarbonate concentration of the mature leaf protoplasts treated with carbonic anhydrase encapsulated and control liposomes in Example 2;

Fig. 7 is a graph showing the enhancement of photosynthesis by the introduction of carbonic anhydrase into mature leaf protoplasts by the liposome treatment in Example 2.

BEST MODE FOR CARRYING OUT THE INVENTION

In order to increase the photosynthetic efficiency of useful C3 plants in the general field under a low $CO_2$ condition, besides elevating the environmental $CO_2$ concentration, it is conceivable to increase the plant leaf affinity towards $CO_2$ , i.e., to enlarge the pipe of the intracellular $CO_2$ supply and transport process (or to reduce the resistance for $CO_2$ transport) from the surface of the photosynthetic tissue (or leaf) to the site of $CO_2$ fixation in the cell.

The present $CO_2$ transporting process of the intracellularly penetrated $CO_2$ in photosynthetic cells of C3 plants towards the $CO_2$ fixation site (RuBP carboxylase) can be reviewed as follows; That is, when the "$CO_2$" penetrates, through cytoplasmic membrane from the outside to the cytosol, the $CO_2$ takes the "$CO_2$

3

EP 0 190 362 B1

form", instead of "$HCO_3^-$" or $H_2CO_3$ and dissolves into the cytosolic stream in the form of an ionic type "$HCO_3^-$" or "$CO_2$" according to the pH of the medium. Both forms of the carbon dioxide and bicarbonate diffuse toward the $CO_2$ consuming site, i.e., chloroplasts, according to the concentration gradient in the cell. In the form of "$CO_2$", inorganic carbon penetrates two layers of the chloroplast envelope into the stroma and dissolves as "$CO_2$" and "$HCO_3^-$" in a certain ratio according to the pH in the chloroplasts.

During photosynthesis, the pH in chloroplasts is calculated to be as high as about 8.0, and in cytosol about 7.0. Henderson-Hasselbach's equation leads to the calculation that the $HCO_3^-/CO_2$ ratio is around 40 in chloroplasts and the ratio is about 4 in cytosol.

It is remarkable that most of the inorganic carbon is present in the form of "$HCO_3^-$" rather than in the form of $CO_2$. "$CO_2$" is the substrate form for the photosynthetic enzyme ribulose-1,5-bisphosphate carboxylase (RuBP CX) instead of "$HCO_3^-$". The equilibration rate of the hydration reaction of $CO_2$ and dehydration of $HCO_3^-$ (i.e., $CO_2 + H_2O \rightleftharpoons HCO_3^- + H^+$) is slower than the photosynthetic $CO_2$ fixation rate in the absence of carbonic anhydrase. Thus, the suboptimum level of carbonic anhydrase in the system might lead to a limiting of the supply of substrate $CO_2$ for the carboxylation enzyme RuBP carboxylase during light saturated photosynthesis.

Fortunately, the carbonic anhydrase activity is found to be predominantly localized in the chloroplasts in C3 plants. The $CO_2$ penetrated in a chloroplast will be readily equilibrated with two forms of $HCO_3^-$ and $CO_2$ in the actual cells. This will initiate a reduction of the local $CO_2$ concentration inside the chloroplast envelope and lead to an increase in the gap in $CO_2$ levels between the outer and inner microcosmos. Accordingly, this will lead to an enhancement of the diffusion rate through chloroplast membranes, and all of the steps described above contribute to an increase in the supply of substrate $CO_2$ toward RuBP carboxylase, and of simultaneous inhibitor $CO_2$ RuBP oxygenase (Okabe K. et al. FEBS Lett. 114, 142-144 (1980)). However, it is apparent that the scarceness of the carbonic anhydrase activity in the cytosol of a C3 plant cell causes the limitation in the rate for the supply of "$CO_2$" (the form of penetration through the chloroplast membrane) from "$HCO_3^-$" at the surface of chloroplasts membranes and also the reduction in the diffusion rate from outside of the cell to the cytoplasm because of the decrease in the $CO_2$ concentration gap between the outer and inner cell membrane caused by the slow equilibration from entered $CO_2$ to $HCO_3^-$ in the micro cytosolic environment. Thus, it is easily understood that the layer of cytosol is the rate limiting step in the photosynthetic $CO_2$ transporting process.

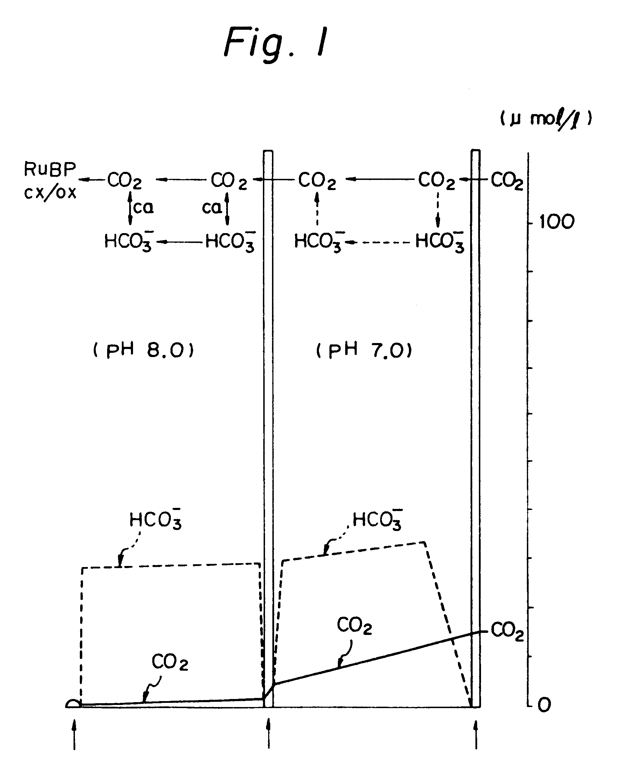

Figure 1 is a schematic diagram showing the intracellular concentration gradient of "$CO_2$" and "$HCO_3^-$" during the photosynthesis of a C3 plant, as mentioned above. In the figure, Ca indicates the location of the carbonic anhydrase.

Consequently, the inventors thought to introduce a carbonic anhydrase activity into the cytosolic layer (including cytomembrane) of the living C3 plant cells, expecting an increase in the photosynthetic $CO_2$ fixation capacity under the environmental $CO_2$ limiting condition.

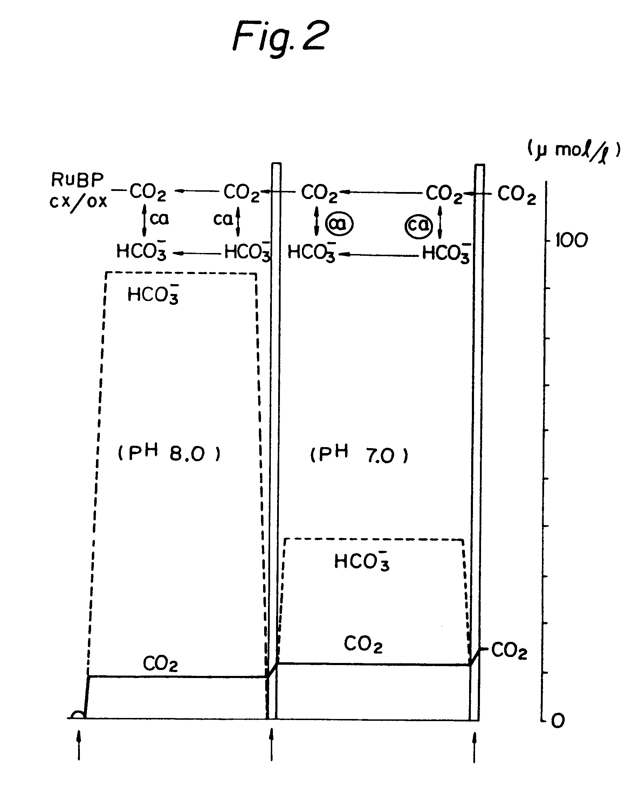

On the basis of the above reasoning, the inventors made an extensive study into the introduction of carbonic anhydrase activity experimentally into cytosol and/or cytomembrane, and thus improved the rate limiting step in photosynthetic transport of inorganic $CO_2$ from the outer surface of a cell to the $CO_2$ fixation site, i.e., carbonic anhydrase was first introduced into liposomes and the resultant liposomes were fused with protoplasts of the C3 plant. The photosynthetic activities of these protoplasts were measured, and showed a significant enhancement under a lower $CO_2$ concentration in the medium than that of the control liposome treated protoplasts (carbonic anhydrase induction effect). In this experiment, the liposome mediated introduction of carbonic anhydrase showed an activity distribution not only in cytosolic but also in cytomembrane fractions. This evidence supports the concept that the rate limiting step is really at the site of the cytosol and/or cytomembrane. It can be understood that the increase in the total inorganic $CO_2$ level in the process of $CO_2$ transport is achieved by the introduction of carbonic anhydrase into the cellular space, as shown in Fig. 2.

From the foregoing, the introduction of a carbonic anhydrase activity into the cytosolic and/or cytomembranous layer is an apparently promising lead to an improvement of the photosynthetic properties in C3 plants, and opens the way to the application thereof to breeding programs for C3 plants. Figure 2 is a schematic diagram showing the concentration gradient of the substrate "$CO_2$" in a photosynthesis of an improved C3 plant. The mechanism of the improved metabolic activity is explained as follows:

(1) The cytosolic existence of carbonic anhydrase activity mediates an immediate equilibration of transported $CO_2$ from extracellular space with $HCO_3^-$ ion according to the pH (ca. pH 7.0 in light). Thus, the inner side of the cellular membrane holds the micro-environmental level of "$CO_2$" in the engineered cells lower than the control present C3 plant cells. Accordingly, the greater gap in $CO_2$ concentration levels would lead to a stimulation of the $CO_2$ uptake by the cell.

(2) The presence of a sufficient level of cytosolic carbonic anhydrase activity also leads to an increase in

4

the supply of $CO_2$ (instead of $HCO_3^-$) to chloroplasts from the cytoplasm, which accelerates finally the total supply of $CO_2$ ($HCO_3^-$ + $CO_2$).

(3) Because of the reasons given in (1) and (2), the substrate $CO_2$ level for $CO_2$ fixation is consequently elevated in the chloroplasts.

In the Fig. 2, Ca indicates the carbonic anhydrase.

Although the above mentioned cellular engineering method consisted of processes of entrapping the enzyme carbonic anhydrase into liposomes, of fusing them with the enzymatically naked host protoplasts, and of the immobilization of the host protoplasts/cells, for breeding purposes, a genetic intracellular expression of the carbonic anhydrase activity is required and can be easily achieved by well known techniques of genetic cellular engineering.

Thus, the present invention covers the claims of the genetical cellular engineering methods which enable the host cells to express a carbonic anhydrase activity in each cytosol and/or cytomembrane in photosynthetic cells in C3 plants after the introduction of the required genetic information relating to the gene expression thereof and the required amino acid sequences into protoplasts, cultured cells or apical meristem cells.

A more detailed explanation of the above method is given in the following examples.

1. Process for carbonic anhydrase gene cloning

(1) Carbonic anhydrase of the C3 plant soybean is, though chloroplast protein, coded in nuclear DNA and synthesized in the cytoplasmic space, transported into chloroplasts, and becomes, a smaller subunit peptide (mature size). The activity of the soybean etiolated leaves is induced by light illumination in accordance with the greening process. Simultaneously, the messenger RNA level thereof increases in the leaves, and thus the complementary DNA (cDNA) library is constructed using plasmids according to the method of Heidecker, G. & J. Messung [Nucleic Acids Research 11, 4891-4901 (1983)]. Screening of the transformants which produce the carbonic anhydrase peptide is carried out for nitrocellulose membranes on which colonies are grown or blotted using the first antibody and then a peroxidase conjugated antibody.

Recombinant plasmids (pSCA series) are further selected based on the criteria of the possibility of expressing CA activity in size, and are used as the passenger gene for plant host vector cartridges.

Genomic carbonic anhydrase genes are also selected using cDNA as the probe, mentioned above, from genomic DNA libraries, constructed from restriction enzyme digestion products of genom DNA and are used as passenger genes for plant host vectors.

However, the cloned carbonic anhydrase genes are required to be processed in the position of base sequences corresponding to the required peptide sequences for penetration thereof into chloroplasts during the above mentioned procedure.

(2) Where the above enzyme carbonic anhydrase gene or carbonic anhydrase cDNA are derived from green algae, Chlamydomonas, in which most of the carbonic anhydrase activity is localized on the cell surface they can be used as passenger genes directly for the plant host vectors, without special gene engineering, since the carbonic anhydrase is encoded in nuclear DNA [Toguri, Yang, Okabe, Miyachi :FEBS Lett.170:117-120(1984)],synthesized in cytosol, and moves to the cell surface.

(3) Also, where the above carbonic anhydrase gene or its cDNA are derived from a C4 plant (such as maize), which carries the enzymic activity mainly in cytosol, they can be used as a passenger gene for plant host vectors without any special processing for the same reasons as mentioned above in (2).

2. Procedures for introduction of carbonic anhydrase gene into plant cells

(1) The introduction of the carbonic anhydrase gene into plant cells using the Ti system of Agrobacterium tumefaciens is carried out according to conventional procedures, which consist of three steps, such as the introduction of carbonic anhydrase gene into intermediate vector, the homologous recombination of the resultant intermediate vector with Ti plasmid, and the transformation of the host plant by the Agrobacterium which carry the resultant recombinant Ti plasmid.[L.Herrera-estrela et al : Nature 310, 115-120 (1984)] [L.Herrera-Estrela et al. :Nature 303, 209-213 (1983)].

(2) The introduction of the above gene into the intermediate vector consists of the selection of broad host ranged plasmid [such as pRK 290, Ditta et al. Proc.Natl.Acad.Sci.USA77:7347-7351 (1980)]. The T region of the Ti plasmid is exercised and introduced into the vector with an antibiotics resistant marker gene(s), the above gene, and a poly $A^+$ RNA signal after the above gene sequence, and placed between the proper promotor and terminator as well as a nopalin synthesis gene fragment (right border sequence of T-DNA).

(3) Homologous recombination of the intermediate vector with the Ti plasmid. The above mentioned intermediate vector is introduced into agrobacterium harboring a Ti plasmid (for example, pTi36S3) through a helper plasmid and the homologous recombinants having a T region of an intermediate vector are selected.

(4) The introduction of a recombinant Ti plasmid harbored in agrobacterium into plant cells is carried out through cultured cells or protoplast, or leaf or stem, by the direct transfection method. Screening of the transformants are carried out by determining the antibiotics tolerance and the above enzyme activity.

Through the above procedure, transformants having the above enzyme activity in cytosol and/or cytomembrane can be selected. Ti plasmids or their derivatives allow the regenerates to flower and the above properties can be transmitted through seeds. Thus, the present invention is also feasible in the plant breeding field.

Typical applications of the present invention for a C3 plant have been described above, however, this invention is not limited to those described. The invention is also applicable for the activation of several metabolic cycles where the $HCO_3{}^-$ level is rate limiting in the above described various cells. This invention is also applicable, for the improvement of secondary metabolites production by immobilized cells (bioreactor), because the newly introduced carbonic anhydrase activity might enhance the supply rate of $HCO_3{}^-$ to phosphoenol pyruvate carboxylase (catalyst of C1-C3 carboxylation), which plays a key role in the biosynthetic pathways of the above mentioned secondary metabolites, such as amino acids.

Examples

This invention is set out in more detail in the following examples. Of course, the claims of this invention are not restricted by these examples.

Example 1

Enhancement of photosynthetic $CO_2$ fixation by the introduction of carbonic anhydrase into tomato cotyledonousprotoplasts

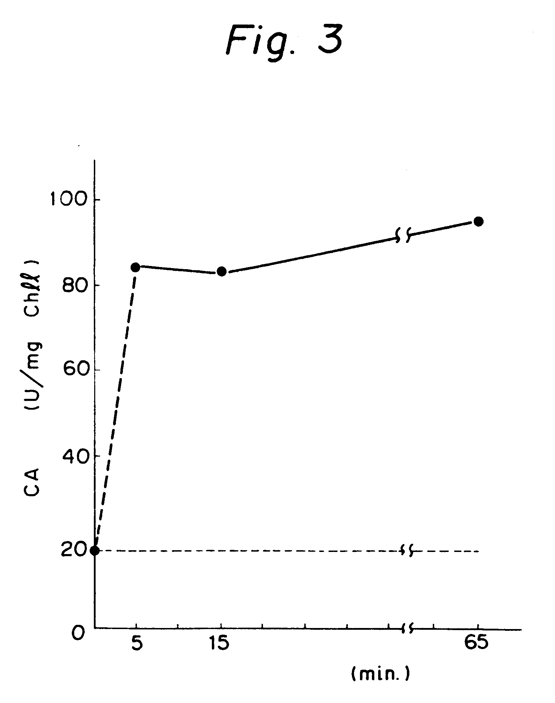

Intact protoplasts were isolated and purified from cotyledons of tomato (Lycoperisicom esculentum) according to the procedure shown in the flow chart of Table 1. The preparation of liposomes with or without carbonic anhydrase was carried out according to the method shown in Table 2, using dipalmytoylcholine and stearylamine. Protoplasts having entrapped and non-entrapped carbonic anhydrase were obtained after the fusion procedure shown in Table 3. The time course of the fusion process is shown in Fig. 3.

In Fig. 3, the activities detected in the liposome-treated protoplasts after thorough washing are plotted versus the incubation time for liposomes protoplasts fusion. After washing, the measured carbonic anhydrase activity corresponded to a number of liposome particles as high as 472 per protoplast and the supernatants contained null activity.

After the puncture of treated protoplasts in the isoosmotic condition with chloroplasts by passing them through a 20 m$\mu$ pore size nylon filter, about 26% of the introduced carbonic anhydrase activity was recovered in the cytoplasmic fraction (supernatant) and 74% in the precipitates containing chloroplasts and protoplast membrane. In this experiment, an about 120 times higher level of the intrinsic carbonic anhydrase activity of native protoplast was recovered (Table 4).

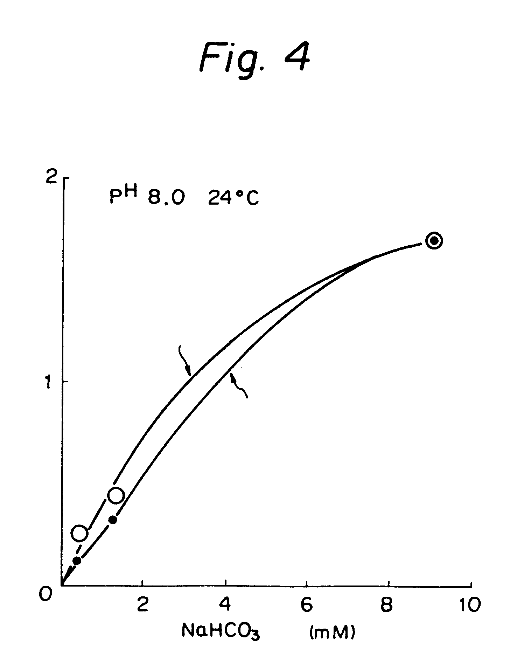

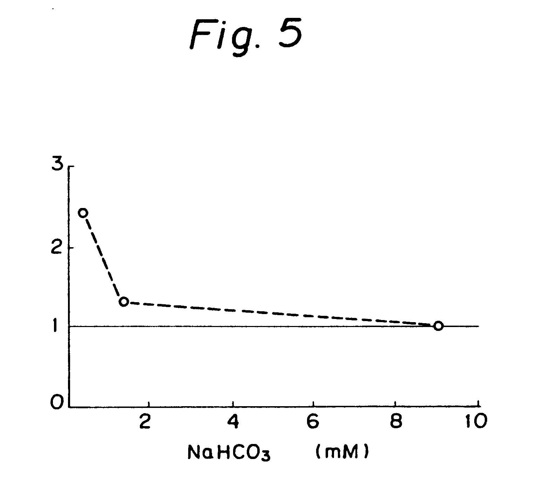

With the protoplasts prepared as mentioned above, the photosynthetic carbon dioxide fixation activity was measured under a light saturated condition (10,000 lux) using $NaH^{14}CO_3$ as the substrate. In each measurement, a carbonic anhydrase solution was added to the reaction medium at the final concentration of 0.5 mg/ml in 0.25 ml, to avoid limiting the supply of $CO_2$ from the medium toward the protoplast membrane surface. The reaction was started after 10 min of preillumination by the addition of sodium bicarbonate ($NaH^{14}CO_3$) at a final concentration of 0.36 mM, 1.25 mM, or 9 mM, and stopped by the addition of acetic acid after 30 sec. The acid stable fixed $^{14}C$ was measured by a liquid scintilation counter. As shown in Fig. 4 and 5, the lower the bicarbonate concentration, the more enhanced the photosynthesis of carbonic anhydrase entrapped protoplasts. The above data suggest that the efficiency of $CO_2$ uptake was increased under photosynthetic limiting $CO_2$ concentration and protoplastic or cellular photosynthetic efficiency can be elevated until the introduced carbonic anhydrase deactivates. It is remarkable that the experiments shown in Fig. 4 and 5, which are the bicarbonate dependent curves of photosynthetic efficiency (relative ratio) of liposome mediated carbonic anhydrase introduced protoplasts and the degree of photosynthetic enhancement (relative ratio) by its introduction, respectively, were designed to be able to clarify the effect of difference(s) of carbonic anhydrase existence only in the intraprotoplastic space.

Table 1 Procedure for preparation of cotyledonous protoplasts

1. Tomato cotyledons were harvested after 14 days growth in watered vermiculite beds under a continuous fluorescent illumination (3,000 lux).
2. The cotyledons were rinsed with distilled water.
3. The cotyledons were cut into 0.5-1.0 x 2 mm strips.
4. The cotyledon strips were incubated in a mixture of 1% cellulose and 0.25% Macerozyme in 0.4 M mannitol for 4 hrs, passed through a 58 m$\mu$ pore size nylon filter, and the filtrate collected.
5. The filtrate was centrifuged at 50 x g (2,000 rpm) for 5 min to sediment the protoplasts, the supernatant removed, and resuspended the protoplasts with 1 ml of 0.4 M mannitol solution. The protoplasts suspension then centrifuged, after layering the top at a stepwise gradient of 10, 20, and 30% of a Ficol 400 layer, at 2,000 rpm for 5 min to recover intact the protoplasts band.
6. The protoplasts solution was diluted with a 0.4 M mannitol solution and the protoplasts recovered by centrifuging at 800 rpm for 5 min.
7. The protoplasts were then washed with the above solution and thus the protoplasts were purified.
8. They were then kept on ice until used for an experiment.

Table 2 Procedure for preparation of liposome

1. Dipalmytoylphosphatidylcholine (2.5 mg) and stearylamine (0.27 mg) was dissolved in chloroform (1 ml) and the solvent dried under a stream of nitrogen in a 20 ml sterile egg-type flask.
2. The lipids were dissolved into 0.6 ml of ice cold ethylether and mixed with 0.15 ml of the carbonic anhydrase solution consisting of 8 mg carbonic anhydrase, 0.5 M sorbitol, and 1/10 PBS (pH 7.8). For the control liposomes, carbonic anhydrase is omitted in this step.
3. The mixture was emulsified in a nitrogen atmosphere with a sonicator to obtain reverse phase micelles.
4. After standing on ice for a while, the ether was dried under a stream of nitrogen to obtain large reverse phase micelle fusion bodies.
5. The liposomes were solubilized in a 0.5 M sorbitol solution and centrifuged at 16,000 rpm for 4 min at 4°C.
6. Step 5 was repeated three times and the complete clearance of carbonic anhydrase activity in the last washing supernatant was confirmed.
7. The washed liposomes were resuspended in 1 ml of a 0.5 M sorbitol solution.

Table 3 Procedures for fusion of protoplasts and liposomes

1. The protoplasts suspension prepared according to the procedures described in Table 1 was mixed with a 200 times larger number of liposomes prepared as described in Table 2.
2. The reaction mixture was left to stand at room temperature.
3. The protoplasts were then recovered by centrifugation at 2,000 rpm for 5 min.
4. The fused protoplasts were washed three times with centrifugation in 0.4 M mannitol (pH 7.0), resuspended and kept on ice until used for an experiment.

## Table 4   Cellular distribution of carbonic anhydrase

### introduced into protoplasts

| | Carbonic anhydrase activity (U/ml extract) | Chlorophyll (µg/ml extract) |
|---|---|---|
| Protoplasts only: | | |
| Supernatant (cytosol) | 0.0 | 0.0 |
| Precipitate (mainly chloroplasts) | 0.5 | 26.9 |
| Protoplasts + liposome (control): | | |
| Supernatant (cytosol) | 0.0 | 0.0 |
| Precipitate (mainly chloroplasts) | 0.5 | 30.6 |
| Protoplasts + liposome (carbonic anhydrase): | | |
| Supernatant (cytosol) | 18.7 | 1.5 |
| Precipitate (mainly chloroplasts) | 40.6 | 19.1 |

Example 2

Stimulatory effect of introduced carbonic anhydrase on the photosynthetic $O_2$ evolution rate of tomato leaf protoplasts

Photosynthetically highly active protoplasts were prepared from mature leaves of a C3 plant, tomato (Lycoperisicom esculentum), following the procedure of the flow chart shown in Table 5. Liposomes having encapsulated or non-encapsulated bovine carbonic anhydrase were prepared according to the method described in the flow chart shown in Table 6. Protoplasts having entrapped or non-entrapped bovine carbonic anhydrase were prepared by fusion reaction with the above two materials. In this experiment the carbonic anhydrase activity of the introduced protoplasts was about two levels higher (4.63 U/3 x $10^5$ protoplasts) than those of the control liposome treated or control non-treated protoplasts (2.3 U/3 x $10^5$ protoplasts). On average, levels of carbonic anhydrase activity from two to four times higher were introduced into protoplasts with this preparation method.

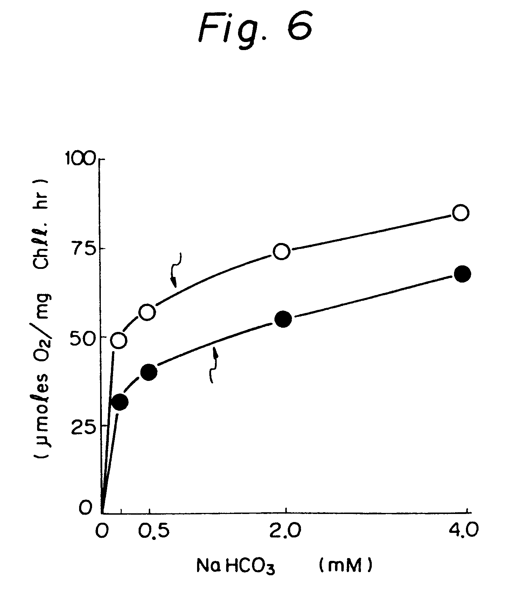

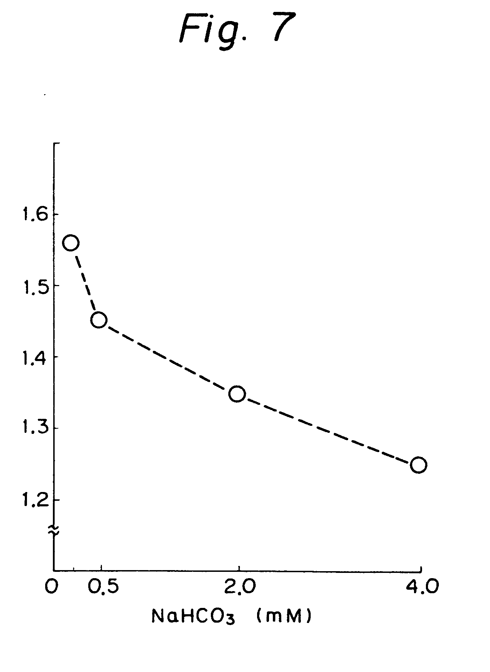

The photosynthetic property of those leaf protoplasts prepared as mentioned above were determined with respect to the relationship between the $O_2$ evolution and substrate bicarbonate concentration, using Rank-Brother oxygen electrodes according to the procedure flow chart shown in Table 8. Both liposomes-treated protoplasts were resuspended in a $CO_2$ freed buffer, placed in the oxygen electrode vessel with the existence of a carbonic anhydrase solution (42 U/ml) to avoid limiting the $CO_2$ supply towards the protoplasts surface and preilluminated until a trace amount of contaminated $CO_2$ dependent on the $O_2$ evolution is ended. At around a 21% $O_2$ partial pressure level, several known levels of substrate bicarbonate were added and a maximum photosynthetic $O_2$ evolution rate was determined and compared. The relationship of the photosynthetic rate and substrate bicarbonate concentration, shown in Fig. 6, exhibits a substrate bicarbonate concentration dependent on the photosynthetic $O_2$ evolution. Especially under a low bicarbonate concentration, the carbonic anhydrase entrapped protoplasts showed a higher photosynthetic rate than the control protoplasts. This enhancement by the carbonic anhydrase introduction into protoplasts was higher in a lower bicarbonate concentration region as shown in Fig. 7.

The intra-protoplastic distribution of the introduced carbonic anhydrase in the above mentioned condition was determined in cytosol fraction and chloroplasts as well as cytomembrane fractions while only trace carbonic anhydrase activity was detected in the cytoplasm of the non-treated and control liposometreated protoplasts.

From the foregoing, the feasibility of this cellular engineering technique that enables the expression of an increased level of carbonic anhydrase activity in the cytosol and/or cytomembrane surface layer of C3 plants is apparent from the view point of the aimed enhancement of $CO_2$ utilization efficiency in photosynthesis under environmental conditions.

Table 5 Procedures for preparation of leaf protoplasts

1. Tomato plants were grown in a growth chamber for 15 days in a mixture of Vermiculite and Venuslight bed with a photoperiod of 14 h/day (2,500-6,000 lux). The first trifoliate leaves were harvested. Lower epidermis of the leaves were peeled off and floated in the 0.5 M manitol solution to allow plasmolysis at room temperature.

2. The leaves were transferred into an enzyme solution (1% Cellulase R-10, 0.01% Pectolyase Y-23, 0.5 M mannitol, pH 7.0) and incubated for 2 hrs in the dark at 37°C to obtain protoplasts.

3. The incubated material was passed through a 67 m$\mu$ mesh nylon filter and the filtrate collected.

4. The protoplast suspension was centrifuged at about 100 x g for 3 min to remove the supernatant enzyme solution and resuspended in a 0.5 M mannitol solution. The protoplasts suspension was centrifuged after placing on the top layer of the 20% (w/w) sucrose solution and bandary intact protoplasts obtained on the 20% sucrose layer.

5. The protoplasts were recovered with a Pasteur pipette, the washing in 0.5 M mannitol solution repeated three times, and the purified protoplasts kept on ice.

Table 6 Procedures for preparation of liposome

1. Phosphatidylcholin (25 mg, Sigma type-E) and stearylamine (2.7 mg) were dissolved into 3 ml of chloroform, the solution transferred into a 50 ml conical flask, and then dried under a stream of nitrogen.

2. The lipids were dissolved into ice cold ethylether (3 ml) and mixed with 1.5 ml of carbonic anhydrase solution (10 mg carbonic anhydrase, 0.5 M mannitol, 0.1 M HEPES, pH 6.2, 0.09% NaCl). (Control liposomes were prepared with the solution without carbonic anhydrase.)

3. The mixture was emulsified in a nitrogen atmosphere with a sonicator to obtain reverse phase micelles.

4. After allowing to stand for 30 min, the flask was placed on a rotary evaporator and the ether dried out by rotation at 150 rpm for 10 min, to obtain fused micelles.

5. The Liposomes were resuspended in a 0.5 M mannitol solution and passed through a 5 m$\mu$ nylon mesh filter and a 1 m$\mu$ Newclearpore filter.

6. The filtrate was ultracentrifuged at 10,000 x g for 10 min at 10°C.

7. The precipitate was washed with a 0.5 M mannitol solution, resuspended in the same solution, and kept on ice.

8. The supernatant of the step 6 was ultracentrifuged for another 3 hrs at 160,000 x g at 10°C, the precipitate washed in 0.6 M mannitol, resuspended in the same solution, and kept on ice.

9. The precipitates obtained in steps 7 or 8 (liposomes) were used for fusion with the protoplasts.

Table 7 Procedures for fusion of protoplasts and liposomes

1. The leaf protoplasts were suspended in a solution of 0.5 M mannitol/10 mM HEPES (pH 6.2) at a concentration of $10^6$/ml and mixed with liposomes (+ or - carbonic anhydrase) suspended in a 0.5 M mannitol solution (0.1 mM lipids) at the ratio of 1:1 on a plastic Petri dish by dropwise mixing to cause the fusion thereof. For the control, the mannitol solution only was used instead of liposomes.

2. After 15 min, the $CaCl_2$ solution was added to the 0.5 M mannitol solution and mixed gently. After 5 min incubation, the same solution was added and mixed.

3. The fusion mixture was spun on the 25% (w/w) sucrose solution at 100 x g for 3 min and leaf protoplasts obtained as a boundary on the 25% sucrose layer.

4. The purified leaf protoplasts were recovered with a Pasteur pipette and resuspended in the same solution. This step was repeated three more times to ensure that the protoplasts were thoroughly washed.

Table 8 Procedures for the determination of photosynthetic oxygen evolution activity

1. Leaf protoplasts (2-3 x $10^5$) were collected by centrifugation at 100 x g for 3 min and kept on ice under a weak light illumination (below 280 lux, for less than 60 min).

2. The leaf protoplasts were resuspended in 1.2 ml of a $CO_2$ freed measuring buffer (0.5 M mannitol, 50 mM HEPES, pH 8.2, 42 U carbonic anhydrase/ml) and the suspension placed in a Clerk type oxygen electrode vessel (Rank-Brothers). The protoplasts were allowed to consume a trace amount of contaminated $CO_2$ in the medium under white light illumination at ca. 10,000 lux and 25°C and a bicarbonate solution added with a microsyringe to obtain 0.2, 0.5, 2.0 and 4.0 mM, consequently. The maximum steady state oxygen evolution rate at each bicarbonate concentration was determined.

3. The oxygen absorption rate in the dark was determined and added to each value obtained in step 2, to estimate the actual photosynthetic oxygen evolution rate.

4. Chlorophyll was extracted by 80% acetone treatment and the content determined spectrophotometrically. The photosynthetic rate on a chlorophyll base was calculated.

## Claims

1. A cellular engineering method for the improvement of the $CO_2$ fixation capacity of C3-plant cells comprising introducing carbonic anhydrase activity into intracellular cytosol and/or cytomembrane.

2. A method as set forth in claim 1, wherein introduction of carbonic anhydrase activity is carried out by incubating liposomes containing carbonic anhydrase with protoplasts of said C3-plant cells.

3. A method as set forth in claim 1, wherein a gene encoding said carbonic anhydrase activity is introduced into C3-plant cells thereby enabling photosynthetic cells of the plant to express carbonic anhydrase activity genetically in the cytosol and/or cytomembrane.

4. A method as set forth in claim 3 wherein the gene encoding said carbonic anhydrase activity is derived from another plant, an animal, an alga or a bacterium.

5. A method as set forth in claim 3 or claim 4 wherein the gene is incorporated into protoplasts, cultured cells or apical meristem cells of a C3-plant, whereafter the transformed cells are incubated and regenerated into plants.

## Revendications

1. Procédé de génie cellulaire pour l'amélioration du pouvoir de fixation de $CO_2$ de cellules de plantes C3, consistant en l'introduction d'activité d'anhydrase carbonique dans le cytosol intracellulaire et/ou la cytomembrane.

2. Procédé selon la revendication 1, dans lequel l'introduction d'activité d'anhydrase carbonique est effectuée par incubation de liposomes contenant de l'anhydrase carbonique avec des protoplastes desdites cellules de plantes C3.

3. Procédé selon la revendication 1, dans lequel un gène codant pour ladite activité d'anhydrase carbonique est introduit dans des cellules de plantes C3, permettant ainsi à des cellules photosynthétiques de la plante d'exprimer génétiquement l'activité d'anhydrase carbonique dans le cytosol et/ou la cytomembrane.

4. Procédé selon la revendication 3, dans lequel le gène codant pour ladite activité d'anhydrase carbonique est tiré d'une autre plante, d'un animal, d'une algue ou d'une bactérie.

5. Procédé selon la revendication 3 ou 4, dans lequel le gène est incorporé dans des protoplastes, dans des cellules cultivées ou dans des cellules de méristème apical d'une plante C3, après quoi les cellules

10

transformées sont mises en incubation et régénérées dans des plantes.

**Patentansprüche**

1. Zellulareinbauverfahren zur Verbesserung des $CO_2$-Fixiervermögens von C3-Pflanzenzellen, bei dem Kohlensäureanhydrase-Aktivität in das intrazellulare Cytosol und/oder die Cytomembran eingeführt wird.

2. Verfahren nach Anspruch 1, bei dem die Einführung der Kohlensäureanhydrase-Aktivität durchgeführt wird, indem man Kohlensäureanhydrase enthaltende Lyposome mit Protoplasten der C3-Pflanzenzellen inkubiert.

3. Verfahren nach Anspruch 1, bei dem ein Gen, das die Kohlensäureanhydrase-Aktivität verschlüsselt, in die C3-Pflanzenzellen eingeführt wird, damit auf diese Weise Photosynthese-Zellen der Pflanze Kohlensäureanhydrase-Aktivität genetisch im Cytosol und/oder der Cytomembran zum Ausdruck bringen können.

4. Verfahren nach Anspruch 3, bei dem das die Kohlensäureanhydrase-Aktivität verschlüssende Gen aus einer anderen Pflanze, einem Tier, einer Alge oder einer Bakterie hergeleitet wird.

5. Verfahren nach Anspruch 3 oder 4, bei dem das Gen in Protoplasten, kultivierte Zellen oder Apical Meristem Zellen einer C3-Pflanze eingearbeitet wird, wonach die umgeformt n Zellen inkubiert und zu Pflanzen regeneriert werden.

Fig. 1

# Fig. 2

# Fig. 3

# Fig. 4

# Fig. 5

EP 0 190 362 B1

# Fig. 6

# *Fig. 7*